# EUROPÄISCHE PATENTSCHRIFT

(11) **EP 1 357 872 B1**
(45) Veröffentlichungstag und Bekanntmachung des Hinweises auf die Patenterteilung: **09.05.2007**
(21) Anmeldenummer: 02711761.3
(22) Anmeldetag: 07.01.2002
(51) Int. Cl.: A61F 13/00, A61F 13/10

(54) **WUNDAUFLAGE, HANDSCHUH UND VERFAHREN ZUM ERSTELLEN EINER WUNDAUFLAGE**
WOUND DRESSING, GLOVE AND METHOD FOR PRODUCTION OF A WOUND DRESSING
PANSEMENT, GANT ET PROCEDE DE FABRICATION D'UN PANSEMENT

(30) Priorität: 07.01.2001 DE 10100304
(43) Veröffentlichungstag der Anmeldung: 05.11.2003
(73) Patentinhaber: Berndt, Erik, 67655 Kaiserslautern (DE); Steffes, Jürgen, 52072 Aachen (DE)
(72) Erfinder: Berndt, Erik, 67655 Kaiserslautern (DE); Steffes, Jürgen, 52072 Aachen (DE)
(74) Vertreter: Bauer, Dirk
(86) Internationale Anmeldenummer: PCT/DE2002/000008
(87) Internationale Veröffentlichungsnummer: WO 2002/053073

(56) Entgegenhaltungen:
- DE-C- 742 041
- GB-A- 465 330
- GB-A- 741 659
- US-A- 2 823 444
- US-A- 4 051 848
- US-A- 4 725 279
- US-A- 5 939 339

## Beschreibung

Die Erfindung betrifft eine Wundauflage aus einem textilen Flächengebilde mit einer ersten Haupterstreckungsrichtung und mit wenigstens einer weiteren Haupterstreckungsrichtung, einen Handschuh zur Auflage auf eine Wunde und ein Verfahren zum Herstellen einer Wundauflage aus einem textilen Flächengebilde.

Insbesondere bei oberflächlich zweitgradigen Hautschäden bzw. Spalthautentnahmestellen sind aus dem Stand der Technik verschiedene Verbandprodukte zur Behandlung derartiger Wunden bekannt.

Beispielsweise werden Spalthautentnahmestellen oft mit Salbengazen (Branolind-Gaze, PVP-Jodgaze, Chlorhexidingaze u. ä.) behandelt, die meist mit einem Schutzverband an die Wunde angelegt werden. Einige Tage nach dem Entfernen eines um die Salbengaze gelegten Schutzverbandes erfolgt eine offene Wundbehandlung, wobei die Gaze meistens hart und unflexibel wird. Dies führt zu erheblichen Schmerzen beim Patienten, insbesondere bei einer durchzuführenden Mobilisation des Patienten.

Nachteilig ist auch, dass die Salbengaze bis zum spontanen Ablösen nach einer erfolgten Reepithelisierung auf dem Defektareal verbleibt, sodass eine zuverlässige Beurteilung des Heilungsverlaufs der Wunde meist nur sehr schwer bzw. nicht möglich ist. Außerdem zeigt sich im Vergleich mit anderen Produkten, dass sich die Reepithelisierungsdauer bei der Anwendung von Salbengazen oft wesentlich verlängert. Insbesondere eine Behandlung von thermischen oder chemischen Hautschäden mit Salben (z.B. Silbersulfadizin) erfordert tägliche Verbandwechsel, die für den Patienten meist sehr schmerzhaft sind, da die Salbenreste mechanisch vom Wundgrund der Wunde entfernt werden müssen.

Zur Wundbehandlung sind auch hydrokolloide Verbände (z.B. Varihaesive) oder Folienverbände (z.B. Opsite-Folie) bekannt, die sich im Vergleich zu den Salbenverbänden unter anderem durch ein deutlich niedrigeres für den Patienten fühlbares Schmerzniveau auszeichnen. Bei der Verwendung von derartigen hydrokolloiden Verbänden bildet sich eine Flüssigkeits-/Hydrogelblase (bei Folienverbänden nur Wundsekret) zwischen dem Wundgrund und der Wundauflage. Diese Verbände müssen im Durchschnitt alle 5 Tage gewechselt werden und erlauben in der Zwischenzeit keine ausreichende Wundbeurteilung. Auch der Einsatz an ungünstigen Körperlokalisationen (z.B. Übergang Bein/Becken) erweist sich als äußerst schwierig und ist daher nur eingeschränkt möglich, da sich an ungünstigen Körperstellen auf Grund mangelnder Elastizität die Hydrogelplatten vom Körper ablösen und häufig ein Gemisch aus Hydrogel und Wundsekret aus dem Bereich der hydrokolloiden Verbände ausläuft. Zusätzlich ist zum Einsatz von hydrokolloiden Verbänden eine großflächige Überlappung mit umliegendem gesundem Gewebe notwendig.

Des Weiteren ist aus der Druckschrift US 4,725,279 eine Polyamidtextilstruktur unter dem Namen "Biobrane" bekannt. Hierbei erfolgt eine Behandlung nach einer Wundreinigung durch ein Fixieren des "Biobrane" im Zusammenhang mit einer Anlage eines Schutzverbandes. Dieser Schutzverband wird im weiteren Behandlungsverlauf entfernt, wodurch eine offene Wundbehandlung durchgeführt werden kann. Die Polyamidtextilstruktur verbleibt adhärent bis zur vollständigen Abheilung auf der Wunde und löst sich dann spontan ab. Durch die Elastizität der Polyamidtextilstruktur und das Fehlen von weiteren Verbandwechseln wird von Seiten der Patienten bei der Behandlung mit "Biobrane" über wenig Schmerzen geklagt.

Nachteilig ist auch, dass bei einer Wundinfektion die Grenzfläche Wunde, Wundauflage mit hydrophilen Lokaltopika nicht oder nur unzureichend erreicht werden kann, da die dem Körper abgewandte Seite der Wundauflage meist mit Silikon überzogen ist.

Auch sind Wundverbände bekannt, die aus mehreren übereinander angeordneten Komponenten hergestellt sind. Beispielsweise ist in der Offenlegungsschrift DE 27 088 22 ein Wundverband beschrieben, der aus mehreren Schichten gewirktem Material sowie einer äußeren Schutzsicht hergestellt ist. Zwar hat dieser Wundverband gute Dehneigenschaften, jedoch unterscheiden sich die Dehneigenschaften des Wundverbandes in unterschiedlichen Dehnrichtungen ganz erheblich voneinander, so dass dieser Wundverband nur unzureichend den Körperbewegungen eines Patienten folgen kann.

Im Übrigen betrifft dieser Nachteil ebenso einige der schon erwähnten, aus dem Stand der Technik bekannten übrigen Wundverbände.

Offensichtlich ist es bis heute nicht gelungen, einen Wundverband bereitzustellen, der in nahezu allen Richtungen, insbesondere in den Hauptdehnrichtungen eines Wundverbandes, im wesentlichen gleiche Dehneigenschaften aufweist, die insbesondere mit der Haut eines Patienten im wesentlichen übereinstimmen oder darüber liegen.

Die Aufgabe der Erfindung besteht darin, gattungsgemäße Wundverbände weiterzuentwickeln und einen Wundverband bereitzustellen, der eine einfache, schmerzarme und an den derzeit bekannten Anforderungen der physiologischen Wundheilung orientierte Behandlung von Hautschäden ermöglicht.

Die Aufgabe der Erfindung wird gelöst von einer Wundauflage der im unabhängigen Anspruch 1 definierten Art.

Ein Gewirk hat den Vorteil, dass es bei einem Zurechtschneiden der Wundauflage nicht so leicht aufribbelt wie vergleichbare Materialien.

Es versteht sich, dass das textile Flächengebilde aus Natur- oder Synthetik-Fasern und/oder aus Natur- oder Synthetikfäden hergestellt sein kann.

Es versteht sich des Weiteren, dass das textile Flächengebilde auch aus einer Kombination von verschiedenartigen Fasern bzw. von verschiedenartigen Fäden hergestellt werden kann.

Im wesentlichen können zum Herstellen des textilen Flächengebildes alle denkbaren Materialien herangezogen werden, die von einem Körper gut verträglich angenommen werden bzw. keine oder nur geringe entzündliche Reaktionen hervorrufen. Vorzugsweise wird das textile Flächengebilde mittels Wirken beispielsweise auf Raschelmaschinen oder Kettenwirkmaschinen und mittels Stricken beispielsweise auf Rundstrick- oder Flachstrickmaschinen hergestellt.

Unter dem Begriff "erste Haupterstreckungsrichtung" versteht man vorzugsweise diejenige Richtung, welche sich entlang einer Maschenreihe eines textilen Flächengebildes erstreckt.

Unter dem Begriff "weitere Haupterstreckungsrichtung" versteht man dann vorzugsweise diejenige Richtung, welche sich entlang einer Maschenstäbchenreihe eines textilen Flächengebildes erstreckt und somit von der "ersten Haupterstreckungsrichtung" abweicht. Vorzugsweise verläuft die "weitere Haupterstreckungsrichtung" orthogonal zu der "ersten Haupterstreckungsrichtung". Sie kann aber auch jede beliebig andere Ausrichtung gegenüber der "ersten Haupterstreckungsrichtung" aufweisen.

Besonders vorteilhaft ist es, wenn das textile Flächengebilde wenigstens in zwei der Haupterstreckungsrichtungen im wesentlichen gleiche Dehnungseigenschaften aufweist.

Die Wundauflagen haben eine Längsausrichtung und eine Querausrichtung. Dies beschreibt einen besonders einfachen Aufbau einer Wundauflage, wobei es selbst bei dieser einfachen Ausführungsform der Wundauflage bisher nicht gelungen ist, diese derart zu gestalten, dass die Wundauflage zumindest in einer Hauptdehnrichtung der Längserstreckung und in einer Hauptdehnrichtung einer Quererstreckung im wesentlichen gleiche Dehnungseigenschaft aufweist.

Werden dem textilen Flächengebilde ggf. Elastomerfasern bzw. Elastomerfäden beigegeben, werden zumindest hinsichtlich der Haupterstreckungsrichtungen Dehnungseigenschaften erreicht, die nur noch um ca. 20 % oder weniger voneinander abweichen.

Hierdurch hat die Wundauflage besonders homogene Dehneigenschaften und ist somit besonders gut geeignet unterschiedliche Bewegungen eines Körpers aufzunehmen und ebenfalls zu durchlaufen.

Ein derartiges Merkmal beeinflusst aber unter anderem den Tragekomfort, den Heilungsverlauf sowie das gesamte Behandlungsverfahren positiv, wodurch insbesondere für den Patienten ein wesentlich schmerzfreierer bzw. schmerzverkürzender Heilungsprozess erzielt.

Unter dem Begriff "Dehnungseigenschaft" versteht man im Sinne der Erfindung eine "Dehnung ε", die das Verhältnis einer Längenänderung ΔL = L1 - L0 / L0 im Rahmen einer Höchstzugkraftdehnung beschreibt und beispielsweise als Prozentwert angegeben werden kann.

Um eine Dehnungseigenschaft hinreichend zu erfassen, wird beispielsweise eine Probe mit einer Vorkraft von 0,1 N vorgespannt. Anschließend wird eine kontinuierliche Erhöhung der Kraft vorgenommen, wodurch die Probe immer weiter gedehnt wird. Die Kraft wird hierbei derart lange erhöht, bis sie einen Maximalwert erreicht und unter einer weiteren Dehnung der Probe wieder abfällt. Beispielsweise wird die Probe um 40 mm gedehnt bevor die Höchstzugkraft anliegt.

Um die "Dehnung ε" hinreichend beschreiben und vergleichen zu können, wird hinsichtlich der unterschiedlichen Erstreckungsrichtungen des textilen Flächengebildes eine gleichmäßige Krafteinleitung an diesem vorrausgesetzt.

Der Fachmann erkennt, dass durch die Einführung der Merkmalsangabe "Dehnung ε" ebenfalls ein Wert hinsichtlich einer Elastizität des textilen Flächengebildes beschrieben werden kann.

Es ist von Vorteil, wenn das textile Flächengebilde zumindest in Richtung einer der Haupterstreckungsrichtungen eine Dehnung ε von unter 700 %, vorzugsweise von unter 500 %, aufweist. Es hat sich gezeigt, dass eine derartige Dehnung ε in jedem Falle ausreicht, sich entsprechend einer Gewebedehnung einer Körperoberfläche zu verhalten. Eine weitergehende Erhöhung der Dehnung ε bringt nahezu keinen weiteren Vorteil, sondern erschwert nur die Herstellung des textilen Flächengebildes und macht dieses hinsichtlich der Herstellung um so teurer.

Besonders vorteilhaft ist es, wenn das textile Flächengebilde zumindest in Richtung einer der Haupterstreckungsrichtungen eine Dehnung ε von mehr als 30 %, vorzugsweise von mehr als 50 %, aufweist. Insbesondere im Hinblick darauf, dass eine mit dem textilen Flächengebilde zu behandelnde Körperoberfläche eine Gewebedehnung ε von etwa 50 % aufweist, ist es besonders günstig, wenn die Dehnung ε des textilen Flächengebildes die Dehnung ε des Körpers übersteigt, bzw. zumindest annähernd aufweist.

Eine vorteilhafte Ausführungsvariante sieht vor, dass die Dehnung ε einen Wert zwischen 60 % und 400 %, vorzugsweise einen Wert zwischen 100 % und 300 %, aufweist. Es hat sich gezeigt, dass eine Dehnung ε in einem Intervall zwischen 100 % und 300 % besonders gut für die Wundauflage geeignet ist. Eine Wundauflage mit einer derartigen Dehnung ε schmiegt sich besonders vorteilhaft an eine abzudeckende Wunde an und folgt problemlos nahezu jeder Bewegung des Patienten. Hierdurch verringern sich unter anderem die Schmerzen des Patienten wesentlich, und das Ausheilen der Wunde kann schneller und problemloser verlaufen als bei vergleichbaren Wundauflagen aus dem Stand der Technik.

Ein Einstellen von elastischen Eigenschaften der Wundauflage kann beispielsweise durch eine geeignete Bindung des textilen Flächengebildes und/oder durch eine Temperaturnachbehandlung des textilen Flächengebildes erreicht werden. Beispielsweise geschieht diese Nachbehandlung mittels eines Spannrahmens, wobei unter Einfluss einer Temperaturänderung gewünschte Eigenschaften in das textile Flächengebilde fixiert werden können. Hierzu kann das textile Flächengebilde als gespanntes Material oder als loses Material in einen Ofen einfahren und dort verweilen bis sich die gewünschten Eigenschaften im Material fixiert haben.

Die Wundauflage ist baulich besonders einfach gestaltet, wenn das textile Flächengebilde einlagig ausgebildet ist. Ein weiterer Vorteil eines einlagig ausgebildeten textilen Flächengebildes ist, dass es sich besonders gut an einen Körper anschmiegt.

Um unter anderem eine möglichst hohe Flexibilität bzw. Elastizität der Wundauflage zu gewährleisten, ist es vorteilhaft, wenn die Wundauflage eine Dicke von weniger als 6 mm, vorzugsweise eine Dicke von weniger als 2 mm, aufweist.

Um eine gewisse Festigkeit aufzuweisen, die es ermöglicht, dass die Wundauflage beispielsweise beim Abnehmen von einer Wunde eine genügend hohe Festigkeit aufweist und nicht so leicht zerreißt, ist es vorteilhaft, wenn die Wundauflage eine Dicke von mehr als 0,05 mm, vorzugsweise von mehr als 0,1 mm, aufweist.

Insbesondere wird durch die vorhergehend beschriebenen Dicken des textilen Flächengebildes eine relativ große Eigenbewegung des Patienten zugelassen, die eine krankengymnastische Übungsbehandlung ermöglicht. Unter anderem ist hierdurch eine Kontrakturprophylaxe unmittelbar ab Behandlungsbeginn möglich.

Eine weitere Ausführungsvariante sieht vor, dass die Wundauflage Poren aufweist, die einen Durchmesser von weniger als 2 mm, vorzugsweise einen Durchmesser von weniger als 0,3 mm, aufweisen.

Es ist vorteilhaft, wenn die Poren einen Durchmesser von mehr als 0,02 mm, vorzugsweise einen Durchmesser von mehr als 0,08 mm, aufweisen.

Mittels derartiger Poren wird eine vorteilhafte Porosität geschaffen, die unter anderem der Wundauflage eine gute Permeabilität verleiht, die sich wiederum in vielseitiger Hinsicht vorteilhaft auf eine Wundbehandlung auswirkt. Die gute bzw. homogene Permeabilität wird insbesondere durch die Struktur des textilen Flächengebildes der Wundauflage hervorgerufen.

Mittels der Permeabilität wird eine semiokklusive Wundauflage geschaffen, sodass ein Gas- und Flüssigkeitsaustausch zwischen der Wunde und deren Umgebung stattfinden kann. Hierdurch wird die natürliche Funktion der Haut hinsichtlich eines Gasaustausches hervorragend imitiert.

Dies bedeutet, dass eine feuchte Wunde im Gegensatz zu einer Behandlung mit perforierten Wundverbänden besonders gleichmäßig trocknen kann.

Weiter vorteilhaft ist es, dass die Poren eine hervorragende Adhärenz gegenüber einem Defektareal ermöglichen, da insbesondere Wundsekret in die bzw. durch die Poren wandert und dort verklebt. Hierdurch erhält die Wundauflage eine besonders gute Verbindung gegenüber der Wunde bzw. gegenüber dem Defektareal.

Weiter vorteilhaft ist es, dass eine größer anfallende Menge an Wundsekret durch die Poren abfließen kann.

Wie eingangs schon erwähnt, eignet sich zur Herstellung des textilen Flächengebildes eine Vielzahl an Materialien, wobei das textile Flächengebilde jedoch wenigstens teilweise Seide aufweist. Seide eignet sich besonders gut dazu, ein textiles Flächengebilde als Wundauflage herzustellen, da sich mit der Seide die vorhergehend beschriebenen Dehnungseigenschaften besonders gut erzielen lassen.

Außerdem hat Seide eine gute Feuchtigkeitsaufnahme. Auch kann Seide beispielweise Alkaliene und mineralische Säuren unter Bildung von Salzen aufnahmen.

Eine besonders bevorzugte Ausführungsvariante sieht vor, dass das textile Flächengebilde mehr als 30 % Seide, vorzugsweise mehr als 70 % Seide, aufweist. Ein hoher prozentualer Anteil an Seide ermöglicht eine besonders gute Wundbehandlung, da sie über besonders hohe Feuchtigkeitsaufnahmeeigenschaften verfügt.

Insbesondere durch den Einsatz von Seide bzw. durch den Aufbau des textilen Flächengebildes können bei einem Infekt eine Feuchtigkeitsbildung und/oder eine Blasenbildung an der Wunde von außen festgestellt werden, ohne die Wundauflage von dem Defektareal entfernen zu müssen. Hierdurch wird ein unnötiges Nachschauen im Falle einer gut verlaufenden Wundabheilung unnötig, sodass der Heilungsvorgang ungestört fortgesetzt werden kann.

Des Weiteren hat Seide eine gute Wasser- und Gaspermeabilität, sodass hierdurch die Möglichkeit einer externen Wundbeeinflussung durch hydrophile Lösungen zur Wundbehandlung (z. B. PVP-Jodlösung, Lavasept-Lösungen) besteht. Dies ist insbesondere bei Wundauflagen mit einer nicht permeablen Außenbeschichtung nicht möglich. Insbesondere wird eine Diagnose hinsichtlich der Wunde durch den Aufbau des textilen Flächengebildes ermöglicht.

Es versteht sich, dass hinsichtlich der Verwendung von Seide bevorzugt Naturseide zum Einsatz gelangt, wie beispielsweise die Maulbeerseide oder die Tussahseide.

Des Weiteren weist die Seide bzw. das textile Flächengebilde auf der Grundlage eines Gewirkes eine besonders gute Zuschnittfähigkeit auf, was insbesondere bei einem intraoperativen Zuschnitt von Vorteil ist. Auch ein Reinigen von Seide ist besonders einfach.

Besonders vorteilhaft ist es, wenn das textile Flächengebilde wenigstens einen Grundträger aufweist, welcher einen Proteaseinhibitor aufweist. Beispielsweise ist es möglich, diesen Grundträger mit Wachstumsfaktoren wie beispielsweise Zytokinen zu beladen, sodass hierdurch der Heilungsprozess begünstigt wird. Vorzugsweise liegt der Grundträger in Form eines Fadens vor.

Es versteht sich, dass insbesondere der Grundträger des textilen Flächengebildes gegebenenfalls auch weitere Inhibitoren aufweisen kann, die auch antibiotische oder antibakterielle Zusätze enthalten können.

Um den Heilungsprozess vorteilhaft zu fördern, kann das textile Flächengebilde ein resorbierbares Material aufweisen. Dies kann gegebenenfalls mit dem Defektareal entsprechend reagieren. Vorteilhafter Weise kann mit einem resorbierbaren Material die Permeabilität beeinflusst werden, indem sich durch resorbiertes Material beispielsweise die Porengröße varüert.

Je nach Anwendungsfall ist es vorteilhaft, wenn das textile Flächengebilde wenigstens eine Beschichtung aufweist, sodass die Wundauflage beispielsweise mit Hyaluronsäureesther oder Kollagen versehen werden kann.

Des Weiteren ist es vorteilhaft, wenn das textile Flächengebilde eine Oberfläche aufweist, welche durch Wärme und/oder Druck modifiziert ist.

Ein Modifizieren kann beispielsweise durch ein Kalandrieren, ggf. unter Einbringen einer Temperatur, geschehen. Hierbei gelangt das textile Flächengebilde beispielsweise zwischen zwei Walzen einer Presse, wobei die Walzenoberflächen aus Metall oder elastischen Materialien bestehen können. Hierdurch kann die Oberfläche des textilen Flächengebildes besonders glatt gestaltet werden.

Das vorhergehend beschriebene textile Flächengebilde beeinflusst den Heilungsprozess derart günstig, dass oft schon ab dem dritten Tage eine offene Wundbehandlung möglich ist. Erfordern es die Gegebenheiten, ist es aber auch möglich, die Wundauflage bis zur vollständigen Reepithelisierung auf dem Defektareal zu belassen. Hierbei löst sich die Wundauflage nach Abheilen der Wunde meist spontan von dem Wundgrund der Wunde ab.

Als ideal hat sich ein Entfernen eines Schutzverbandes bis spätestes zum 5. Tag nach dem Behandlungsbeginn erwiesen.

Es hat sich gezeigt, dass nach dem Entfernen des Schutzverbandes und der offenen Wundbeheilung keine weiteren Verbandwechsel notwendig sind.

Dem Fachmann ist ersichtlich, dass das textile Flächengebilde zum Teil mit Nähten durch Näh- und/oder Strickverfahren versehen werden kann. Es ist unter anderem möglich, mehrere derartige textile Flächengebilde miteinander bzw. übereinander mittels Näh- und/oder Strickverfahren zu verbinden. Hierdurch ist es unter anderem möglich, dass die Wundauflage entsprechend des Defektareals exakt angepasst werden kann.

Insbesondere bei großen Defektarealen bzw. Wunden ist zum einen kein weites Überlappen der Wundauflagen untereinander und zum anderen kein weites Überlappen mit einem intakten Gewebe bzw. einer intakten Haut notwendig.

Es versteht sich, dass es vorteilhaft ist, wenn die Wundauflage in Form einer Meterware vorliegt, wobei die Breite vorzugsweise weniger als 4 m beträgt. Hierdurch ist es möglich, nahezu jede nur erdenkliche Form, Größe und Gestalt der Wundabdeckung herzustellen.

Vorzugsweise liegt die erfindungsgemäße Wundauflage als Zuschnitt beispielsweise in unterschiedlichen Anwendungsgrößen sterilisiert und verpackt vor.

Der Fachmann erkennt, dass das textile Flächengebilde sich hervorragend dazu eignet, konfektioniert zu werden. Beispielsweise lässt sich, neben einem direktem Stricken eines Handschuhes, hieraus auch ein Handschuh herstellen.

Dementsprechend wird die Aufgabe der Erfindung von einem Handschuh zur Auflage auf eine Wunde gelöst, wobei der Handschuh aus dem vorhergehend beschriebenen textilen Flächengebilde hergestellt ist. Insbesondere zur Behandlung von Handverbrennungen eignet sich ein Handschuh besonders gut, da dieser die Finger sowie die Handflächen besonders gut umschließt und damit die Defektareale besonders wirksam schützt. Im Gegensatz zu herkömmlichen Wundverbänden sind insbesondere die Finger der Hände wie auch die Hände selbst unmittelbar von Behandlungsbeginn an bewegbar, sodass die Gefahr einer ansonsten häufigen Einsteifung und/oder die Gefahr eines zumindest temporären Funktionsverlustes oder einer zumindest temporären Einschränkung stark reduziert ist. Hierdurch grenzt sich der Handschuh ganz erheblich von bisher bekannten Verbandprodukten ab, da insbesondere Seide ein nicht ruhigstellender Verband ist, der vorteilhafter Weise Eigenbewegungen und krankengymnastische Übungsbehandlungen unmittelbar nach Behandlungsbeginn zulässt.

Des Weiteren wird die Aufgabe der Erfindung von einem Verfahren zum Herstellen einer Wundauflage aus einem vorhergehend beschriebenen textilen Flächengebilde gelöst, wobei das textile Flächengebilde intraoperativ zu einer Wundauflage geformt wird. Zwar ist es von Vorteil, wenn die Gestalt der Wundauflage schon vor einer Behandlung erkannt wird und festliegt, sodass die Wundauflage zum unmittelbaren Einsatz bereitliegt. Ist jedoch das Ausmaß einer Verletzung nicht vorhersehbar, bzw. ergeben sich während einer Behandlung unvorhergesehene Änderungen hinsichtlich eines Defektareals, ist der Chirurg in der Lage, während einer Operation passende Stücke als Wundauflage zurechtzuschneiden.

Das textile Flächengebilde kann aber auch durch Nähen derart geformt werden, dass dadurch eine dreidimensionale Wundauflage entsteht. Beispielsweise ist dies ein Handschuh oder eine sonstig geformte Wundauflage, die um einen Körper oder um einen Körperteil gelegt werden kann.

Weitere Vorteile, Ziele und Eigenschaften vorliegender Erfindung werden anhand vorliegender Zeichnung erläutert, in welcher beispielhaft eine Wundauflage dargestellt ist.

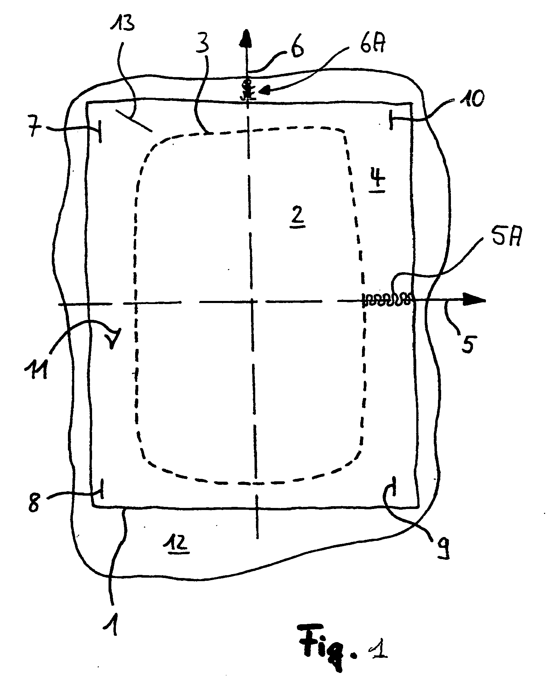

Es zeigt
- die Figur: eine Wundauflage in einer Draufsicht auf einem Defektareal.

Die Wundauflage 1 ist auf ein Defektareal 2 aufgebracht. Der Bereich des Defektareals 2 ist mittels einer Umrandung 3 dargestellt, sodass die Wundauflage 1 in diesem Ausführungsbeispiel auch in einem Bereich 4 um das Defektareal 2 angeordnet ist, sodass die Wundauflage 1 das Defektareal 2 weiträumig überlappt.

Die Wundauflage 1 hat hierbei zwei Haupterstreckungsrichtungen 5 und 6, wobei die Haupterstreckungsrichtung 5 in Richtung einer Maschenreihe 5A der Wundauflage 1 und die Hauptausrichtung 6 orthogonal zu der Maschenreihe 5A der Wundauflage 1 verläuft. Die Hauptausrichtung 6 verläuft dementsprechend entlang der Maschenstäbchen 6A.

Die Wundauflage 1 ist mittels Klammern 7, 8, 9 und 10 im Bereich 4 an einem intakten Hautgewebe 12 fixiert. Dies kann beispielsweise mit Hilfe eines Deltaklammergerätes geschehen.

Die Wundauflage 1 besteht aus Seidenfasern (hier nicht dargestellt) und wird als Gewirk (hier nicht dargestellt) gefertigt. Insbesondere dadurch, dass die Wundauflage 1 als Gewirk gefertigt ist, zeichnet sie sich durch eine gute Schnittfestigkeit aus.

Die Wundauflage 1 ist darüber hinaus derart hergestellt, dass ihre Elastizität in den Haupterstreckungsrichtungen 5 und 6 nahezu identisch ist, was besonders homogene bzw. quasiisotrope Dehnungseigenschaften wenigstens entlang der Haupterstreckungsrichtungen 5 und 6 bewirkt.

Die Wundauflage 1 hat eine Oberfläche 11, welche durch Vorbehandlung mit Wärme und Druck modifiziert ist. Dadurch kann beispielsweise eine besonders glatte Oberfläche erzeugt werden, die ein späteres spontanes Ablösen der Wundauflage 1 von dem Defektareal 2 erleichtert. Des Weiteren werden auf Grund der glatten Oberfläche insbesondere Mikrotraumatisierungen und dadurch bedingte Schmerzen beispielsweise nach Spalthautentnahmen, die unter anderem durch eine Kontraktion von Muskelgruppen unter dem Entnahmeareal im Rahmen einer postoperativen Mobilisation auftreten, vermieden oder zumindest reduziert.

Mittels der Vorbehandlung mit Wärme und Druck wurde hinsichtlich diesem Ausführungsbeispiel das Elastizitätsverhalten an die physiologischen Erfordernisse angepasst.

Die Wundauflage 1 liegt durch eine temporäre Auflage von Kompressen (hier nicht dargestellt) oder Tüchern (hier nicht dargestellt), welche mit vasokonstriktorisch wirkenden Medikamenten (z. B. Suprarenin) getränkt sind, auf dem Defektareal 2 auf.

Die in diesem Ausführungsbeispiel gezeigte geringfügige Überlappung der Wundauflage 1 in dem Bereich 4 ist nicht zwingend erforderlich, was insbesondere bei der Behandlung von Schwerbrandverletzten mit großen Verbrennungsarealen 2 und gegebenenfalls limitierten Spalthautentnahmearealen einen deutlichen Vorteil darstellt. Anschließend erfolgt die Anlage eines Schutzverbandes (hier nicht dargestellt) mit sterilem Verbandmaterial. Der Schutzverband wird nach 3 bis 5 Tagen entfernt und es wird eine offene Wundbehandlung durchgeführt.

Die dem Körper bzw. der Haut 12 abgewandte Seite der Wundauflage 1 trocknet ab, während an der dem Defektareal 2 zugewandten Seite ein feuchtes Wundmilieu entsteht, das der Haut optimale Regenerationsbedingungen liefert. Die Wundauflage 1 wird auf dem Defektareal 2 adhärent angebracht und verbleibt bis zur vollständigen Reepithelisierung auf dem Defektareal 2 angeordnet. Im weiteren Verlauf der Behandlung wird nur noch die bereits von abgeheilten Arealen abgelöste Wundauflage 1 beispielsweise unter Zuhilfenahme einer Schere entfernt.

Der Eintritt einer lokalen Wundinfektion ist durch eine Bildung von Flüssigkeitsansammlungen unter der Wundauflage 1 sowie durch fehlende Adhärenz der Wundauflage 1 mit dem Defektareal 2 einfach zu erkennen. Im Gegensatz zu einer Wundabdeckung mit "Biobrane" besteht die Möglichkeit, die Wundauflage 1 mit hydrophilen Lösungen (z. B. PVP-Jod, Lavasept, Chlorhexidin oder Mefenidacetat) erneut anzufeuchten und somit eine Infektsanierung zu erreichen.

Das Ablösen der Wundauflage 1 vom Defektareal 2 erfolgt spontan. Nach Entfernen des Schutzverbandes (hier nicht dargestellt) und einer offenen Wundbehandlung sind keine weiteren Verbandwechsel notwendig.

Insbesondere durch die im wesentlichen gleiche Dehnungseigenschaft der Wundauflage 1 in den Haupterstreckungsrichtungen 5 und 6 wird eine posttraumatische Bewegungseinschränkung weitestgehend vermieden. Dies trifft insbesondere dann zu, wenn das Defektareal 2 im Bereich einer Hand anzutreffen ist, wobei es aufgrund der Materialeigenschaften der Wundauflage 1 möglich ist, aus dieser einen Handschuh zur Behandlung herzustellen.

Die Wundauflage 1 umfasst ein Fadenmaterial 13 (hier nur exemplarisch dargestellt) aus einem resorbierbaren Polymer, welches gegebenenfalls mit Wachstumsfaktoren getränkt ist. Hierdurch kann eine Wundheilung durch eine kontinuierliche Freisetzung von Zytokinen weiter optimiert und beschleunigt werden.

## Patentansprüche

1. Wundauflage (1) aus einem textilen Flächengebilde mit einer ersten Haupterstreckungsrichtung (5) und mit wenigstens einer weiteren Haupterstreckungsrichtung (6), **dadurch gekennzeichnet, dass**
- das textile Flächengebilde ein Gewirk ist, wobei das Gewirk zumindest entlang der Haupterstreckungsrichtungen (5, 6) jeweils Dehnungseigenschaften aufweist, die weniger als 80 %, vorzugsweise weniger als 50 %, voneinander abweichen, dass
- das Gewirk wenigstens teilweise Seide aufweist, und dass
- die Oberfläche des Gewirks durch Wärme und/oder Druck modifiziert ist.

2. Wundauflage (1) nach Anspruch 1, **dadurch gekennzeichnet, dass** das textile Flächengebilde wenigstens in zwei der Haupterstreckungsrichtungen (5, 6) im wesentlichen gleiche Dehnungseigenschaften aufweist.

3. Wundauflage (1) nach einem der Ansprüche 1 oder 2, **dadurch gekennzeichnet, dass** das textile Flächengebilde zumindest in Richtung einer der Haupterstreckungsrichtungen (5, 6) eine Dehnung ε von unter 700 %, vorzugsweise von unter 500 %, aufweist.

4. Wundauflage (1) nach einem der Ansprüche 1 bis 3, **dadurch gekennzeichnet, dass** das textile Flächengebilde zumindest in Richtung einer der Haupterstreckungsrichtungen (5, 6) eine Dehnung ε von mehr als 30 %, vorzugsweise von mehr als 50 %, aufweist.

5. Wundauflage (1) nach einem der Ansprüche 1 bis 4, **dadurch gekennzeichnet, dass** die Dehnung ε einen Wert zwischen 60 % und 400 %, vorzugsweise einen Wert zwischen 100 % und 300 %, aufweist.

6. Wundauflage (1) nach einem der Ansprüche 1 bis 5, **dadurch gekennzeichnet, dass** das textile Flächengebilde wenigstens mehr als 30 % Seide, vorzugsweise mehr als 70 % Seide, aufweist.

7. Wundauflage (1) nach einem der Ansprüche 1 bis 6, **dadurch gekennzeichnet, dass** das textile Flächengebilde einlagig ausgebildet ist.

8. Wundauflage (1) nach einem der Ansprüche 1 bis 7, **gekennzeichnet durch** eine Dicke von weniger als 6 mm, vorzugsweise weniger als 2 mm.

9. Wundauflage (1) nach einem der Ansprüche 1 bis 8, **gekennzeichnet durch** eine Dicke von mehr als 0,05 mm, vorzugsweise von mehr als 0,1 mm.

10. Wundauflage (1) nach einem der Ansprüche 1 bis 9, **gekennzeichnet durch** Poren, die einen Durchmesser von weniger als 2 mm, vorzugsweise einen Durchmesser von weniger als 0,3 mm aufweisen.

11. Wundauflage (1) nach einem der Ansprüche 1 bis 10, **gekennzeichnet durch** Poren, die einen Durchmesser von mehr als 0,02 mm, vorzugsweise einen Durchmesser von mehr als 0,08 mm, aufweisen.

12. Wundauflage (1) nach einem der Ansprüche 1 bis 11, **dadurch gekennzeichnet, dass** das textile Flächengebilde wenigstens einen Grundträger aufweist, der einen Proteaseinhibitor umfasst.

13. Wundauflage (1) nach einem der Ansprüche 1 bis 12, **dadurch gekennzeichnet, dass** das textile Flächengebilde ein resorbierbares Material (13) aufweist.

14. Wundauflage (1) nach einem der Ansprüche 1 bis 13, **dadurch gekennzeichnet, dass** das textile Flächengebilde wenigstens eine Beschichtung aufweist.

15. Handschuh zur Auflage auf eine Wunde, **gekennzeichnet durch** ein das textile Flächengebilde nach einem der Ansprüche 1 bis 14.

16. Verfahren zum Herstellen einer Wundauflage (1) aus einem textilen Flächengebilde nach einem der Ansprüche 1 bis 14, **dadurch gekennzeichnet, dass** das textile Flächengebilde intraoperativ zu einer Wundauflage (1) geformt wird.

## Claims

1. A wound dressing (1) from a planar textile structure with a first main direction of extension (5) and with at least one further main direction of extension (6), **characterized in that**
- the planar textile structure is a knit fabric, with the knit fabric exhibiting extension properties at least along the main directions of extension (5,6) which vary from each other less than 80 %, preferably less than 50%, and that
- the knit fabric comprises silk at least in part, and that
- the surface of the knit fabric is modified by heat and/or pressure.

2. A wound dressing (1) according to claim 1, **characterized in that** the planar textile structure exhibits essentially identical extension properties in at least in two of the main directions of extension (5, 6).

3. A wound dressing (1) according to one of claims 1 or 2,
**characterized in that** the planar textile structure exhibits an extension ε of less than
700%, preferably of less than 500%, at least in the direction of one of the main directions of extension (5, 6).

4. A wound dressing (1) according to one of claims 1 to 3,
**characterized in that** the planar textile structure exhibits an extension ε of more than 30%, preferably of more than 50%, at least in the direction of one of the main directions of extension (5, 6).

5. A wound dressing (1) according to one of claims 1 to 4,
**characterized in that** the extension ε has a value of between 60% and 400%, preferably a value of between 100% and 300%.

6. A wound dressing (1) according to one of the claims 1 to 5,
**characterized in that** the planar textile structure comprises at least more than 30% of silk, preferably more than 70% of silk.

7. A wound dressing (1) according to one of claims 1 to 6,
**characterized in that** the planar textile structure is formed in one layer.

8. A wound dressing (1) according to one of claims 1 to 7,
**characterized by** a thickness of less than 6 mm, preferably less than 2mm.

9. A wound dressing (1) according to one of claims 1 to 8,
**characterized by** a thickness of more than 0.05 mm, preferably of more than 0.1 mm.

10. A wound dressing (1) according to one of claims 1 to 9,
**characterized by** pores with a diameter of less than 2 mm, preferably a diameter of less than 0.3 mm.

11. Wound dressing (1) according to one of claims 1 to 10,
**characterized by** pores with a diameter of greater than 0.02 mm, preferably a diameter of more than 0.08 mm.

12. A wound dressing (1) according to one of claims 1 to 11,
**characterized in that** the planar textile structure has at least one basic carrier comprising a protease inhibitor.

13. A wound dressing (1) according to one of claims 1 to 12,
**characterized in that** the planar textile structure comprises a resorbent material (13).

14. A wound dressing (1) according to one of claims 1 to 13,
**characterized in that** the planar textile structure has at least one coating.

15. A glove for applying to a wound, **characterized by** a planar textile structure according to one of the claims 1 to 14.

16. A method for producing a wound dressing (1) from a planar textile structure according to one of the claims 1 to 14, **characterized in that** the planar textile structure is formed intraoperatively into a wound dressing (1).

## Revendications

1. Pansement (1) fait d'une structure plane textile avec un sens d'étirement principal (5) et avec au moins un autre sens d'étirement principal (6), **caractérisé en ce que** :
- la structure plane textile est un tricot, lequel tricot possède au moins le long des sens d'étirement principaux (5, 6) des propriétés d'allongement qui diffèrent de moins de 80 %, de préférence de moins de 50 %, **en ce que**
- le tricot contient au moins en partie de la soie, et **en ce que**
- la surface du tricot est modifiée par la chaleur et/ou la pression.

2. Pansement (1) selon la revendication 1, **caractérisé en ce que** la structure plane textile possède des propriétés d'allongement sensiblement identiques au moins dans deux des sens d'étirement principaux (5, 6).

3. Pansement (1) selon l'une des revendications 1 ou 2, **caractérisé en ce que** la structure textile plane présente au moins dans le sens de l'un des sens d'étirement principaux (5, 6) un allongement ε inférieur à 700 %, de préférence inférieur à 500 %.

4. Pansement (1) selon l'une des revendications 1 à 3, **caractérisé en ce que** la structure textile plane présente au moins dans le sens des sens d'étirement principaux (5, 6) un allongement ε supérieur à 30 %, de préférence supérieur à 50 %.

5. Pansement (1) selon l'une des revendications 1 à 4, **caractérisé en ce que** l'allongement ε a une valeur comprise entre 60 % et 400 %, de préférence entre 100 % et 300%.

6. Pansement (1) selon l'une des revendications 1 à 5, **caractérisé en ce que** la structure textile plane contient au moins plus de 30 % de soie, de préférence plus de 70 % de soie.

7. Pansement (1) selon l'une des revendications 1 à 6, **caractérisé en ce que** la structure textile plane est formée d'une seule épaisseur.

8. Pansement (1) selon l'une des revendications 1 à 7, **caractérisé en ce que** son épaisseur est inférieure à 6 mm, de préférence inférieure à 2 mm.

9. Pansement (1) selon l'une des revendications 1 à 8, **caractérisé en ce que** son épaisseur est supérieure à 0,05 mm, de préférence supérieure à 0,1 mm.

10. Pansement (1) selon l'une des revendications 1 à 9, **caractérisé en ce que** ses pores ont un diamètre inférieur à 2 mm, de préférence un diamètre inférieur à 0,3 mm.

11. Pansement (1) selon l'une des revendications 1 à 10, **caractérisé en ce que** ses pores ont un diamètre supérieur à 0,02 mm, de préférence un diamètre supérieur à 0,08 mm.

12. Pansement (1) selon l'une des revendications 1 à 11, **caractérisé en ce que** la structure textile plane possède au moins un support contenant un inhibiteur de protéase.

13. Pansement (1) selon l'une des revendications 1 à 12, **caractérisé en ce que** la structure textile plane comporte un matériau résorbable (13).

14. Pansement (1) selon l'une des revendications 1 à 13, **caractérisé en ce que** la structure textile plane comporte au moins un revêtement.

15. Gant destiné à être posé sur une plaie, **caractérisé en ce qu'**il comporte la structure textile plane selon l'une des revendications 1 à 14.

16. Procédé de fabrication d'un pansement (1) fait d'une structure textile plane selon l'une des revendications 1 à 14, **caractérisé en ce que** la structure textile plane est mise en forme pour produire un pansement (1) pendant une opération.
